(19) Europäisches Patentamt
European Patent Office
Office européen des brevets

(11) Veröffentlichungsnummer : **0 293 462 B1**

(12) **EUROPÄISCHE PATENTSCHRIFT**

(45) Veröffentlichungstag der Patentschrift : **24.04.91 Patentblatt 91/17**

(51) Int. Cl.$^5$ : **C09J 4/02,** C08F 22/00, C08F 255/00, A61K 6/00

(21) Anmeldenummer : **88900684.7**

(22) Anmeldetag : **02.11.87**

(86) Internationale Anmeldenummer : **PCT/EP87/00656**

(87) Internationale Veröffentlichungsnummer : **WO 88/03546 19.05.88 Gazette 88/11**

# (54) DENTALER HAFTVERMITTLER.

(30) Priorität : **06.11.86 CH 4435/86**

(43) Veröffentlichungstag der Anmeldung : **07.12.88 Patentblatt 88/49**

(45) Bekanntmachung des Hinweises auf die Patenterteilung : **24.04.91 Patentblatt 91/17**

(84) Benannte Vertragsstaaten : **AT BE CH DE FR GB IT LI LU NL SE**

(56) Entgegenhaltungen :
**EP-A- 0 196 212**
**DE-A- 1 594 120**
**DE-A- 2 723 339**
**FR-A- 2 342 327**
**GB-A- 2 087 906**
**US-A- 4 113 792**

(73) Patentinhaber : **GDF GESELLSCHAFT FÜR DENTALE FORSCHUNG UND INNOVATIONEN GMBH**
**Dieselstrasse 6**
**W-6365 Rosbach (DE)**

(72) Erfinder : **WAGENKNECHT, Günther**
**Bahnhofstr. 7A**
**W-6363 Echzell (DE)**
Erfinder : **MÜLLER, Jürgen**
**Hauptstr. 93**
**W-6363 Echzell (DE)**

(74) Vertreter : **Lusuardi, Werther Giovanni**
**Dr. Lusuardi AG Kreuzbühlstrasse 8**
**CH-8008 Zürich (CH)**

**EP 0 293 462 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die Erfindung bezieht sich auf einen Haftvermittler basierend auf einem Acryl-, Methacryl- oder Vinyl-Gruppen aufweisenden flüssigen Monomer, Monomerengemisch oder Präpolymer mit einem Zusatz von Acryl- oder Methacrylsäure als Grundgemisch und dessen Verwendung in der Zahntechnik.

Dentale Haftvermittler dienen dazu eine dauerhafte Verbindung zwischen Edel- und Unedelmetallegierungen, bzw. keramischen Materialien einerseits und Dentalkunststoffen anderseits herzustellen.

Das Problem der Verbesserung der Haftung von Kunststoffen auf Dentalprothesenteilen aus Metall oder Keramik ist nicht neu. In jüngster Zeit wurden verstärkt Anstrengungen unternommen, durch Neuentwicklungen langlebige Verbundmöglichkeiten zu erzielen.

Beispielsweise ist aus der US-PS 4.364.731 ein Verfahren bekannt, bei dem die Metalloberfläche im Vakuum mit einer anorganischen Siliziumoxidschicht beschichtet wird, welche durch einen Magnetron-Sprutter von hochreinem Quarzglas abgesondert wird. Ein analoges Verfahren ist in der deutschen Patentschrift DE-C1 34 03 894 beschrieben, bei welchem die $SiO_xC$-Schicht mittels Flammpyrolyse auf das Metall aufgetragen wird. Bei diesen Neuentwicklungen ist allerdings ein erheblicher apparativer Aufwand vonnöten.

Ein weiterer Weg zur Verbesserung der Haftung zwischen Metall und Kunststoff lag in der Erkenntnis, dass Zusätze von Acryl- oder Methacrylsäure zu Monomeren mit radikalisch polymerisierbaren Doppelbindungen ausgezeichnete Adhäsionswerte ergeben (US-A-4 113 792 PASTOR). Allerdings besteht hier der Nachteil, dass die gute Haftung solcher Metall-Kunststoff-Verbundsysteme in kurzer Zeit durch den Einfluss von Feuchtigkeit verloren geht.

Hier will die Erfindung Abhilfe schaffen. Der Erfindung liegt die Aufgabe zugrunde, einen Haftvermittler zu schaffen, mit dem eine dauerhafte Verbindung zwischen Metall und Kunststoff, oder Keramik und Kunststoff, insbesondere zwischen Dentallegierungen und Acrylat-, bzw. Methacrylat-Kunststoffen, wie sie in der Zahntechnik und -medizin üblich sind, erzielbar ist, die den im Mundbereich auftretenden Beanspruchungen (Feuchtigkeit, Temperaturwechsel und mechanische Belastungen) dauerhaft widerstehen kann, ohne Bildung eines Randspaltes zwischen Metall und Kunststoff, bzw. Keramik und Kunststoff.

Die Erfindung löst die gestellte Aufgabe mit einem Haftvermittler, welcher die Merkmale des Anspruchs 1 aufweist. Die durch die Erfindung erreichten Vorteile sind im wesentlichen darin zu sehen, dass dank des erfindungsgemässen Haftvermittlers die Haftung von radikalisch polymerisierbaren Acrylaten, Methacrylaten oder Vinyl-Gruppen aufweisenden flüssigen Monomeren, Monomerengemischen oder Präpolymeren mit Acryl- oder Methacrylsäurezusätzen nur geringen Feuchtigkeitsverlusten unterliegt und somit ein dauerhafter, gleichbleibender Verbund erzielt werden kann.

Die Haftung ist insbesondere dann optimal, wenn dem Grundgemisch beide Zusätze, d.h. sowohl ein halogensulfoniertes Polyolefin als auch ein halogeniertes Acrylat oder Methacrylat beigemischt werden.

Als halogensulfonierte Polyolefine haben sich insbesondere die chlorosulfonierten Polyolefine, vorzugsweise vom Polyäthylen- oder Polypropylen-Typ, bewährt.

Als halogenierte Acrylate oder Methacrylate haben sich insbesondere die fluorierten mono- oder polyfunktionellen Acrylate oder Methacrylate bewährt, welche dem Polymerverbund hydrophobe Eigenschaften verleihen. Speziell gute Ergebnisse wurden mit folgenden fluorierten Monomeren erhalten :

1,1-Dihydro-pentadecafluorooctyl-methacrylat

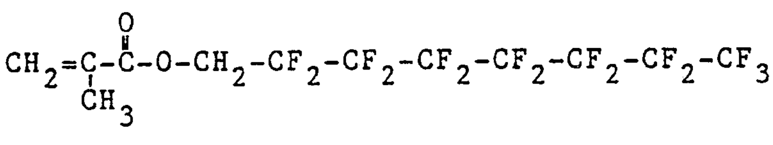

1,1,5-Trihydro-octafluoropentyl-methacrylat

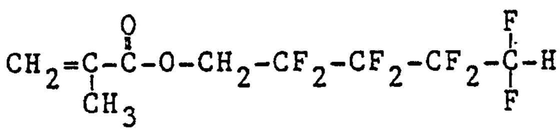

2,2,3,3-Tetrafluoropropyl-methacrylat

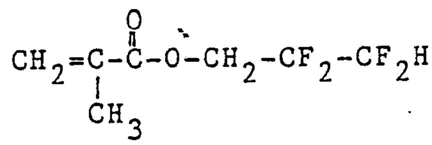

sowie
2,2,3,3,4,4-Hexafluoro-1,5-pentandiol-dimethacrylat

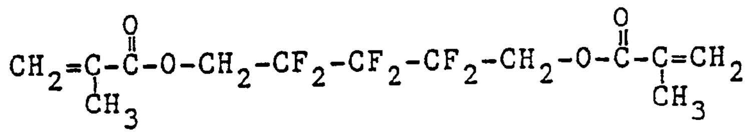

als vernetzendes Monomer.

Nachfolgend wird an Hand von Beispielen die Erfindung näher erläutert.

Beispiel 1 (Stand der Technik) :

Es wurde ein Zweikomponentengemisch aus folgenden Bestandteilen hergestellt :

| | |
|---|---|
| Komponente A : | 70,0 Teile Methylmethacrylat |
| | 18,5 Teile 1,4-Butandioldimethacrylat |
| | 10,0 Teile Methacrylsäure |
| | 1,5 Teile Dibenzoylperoxid |
| Komponente B : | 70,0 Teile Methylmethacrylat |
| | 19,0 Teile 1,4-Butandioldimethacrylat |
| | 10,0 Teile Methacrylsäure |
| | 1,0 Teile N,N'-Dimethyl-p-toluidin |

Durch Vermischen von gleichen Teilen der Komponenten A und B härtet das Material bei Raumtemperatur nach 5 bis 7 Minuten zu einem auf Dentallegierungen kurzfristig fest haftenden Film aus.

Beispiel 2 :

Komponente A :

30 g eines chlorosulfonierten Polyäthylens mit einer Molekulargewichtsverteilung zwischen 20'000 und 40'000, einem Chlorgehalt von 29% und einem Schwefelgehalt von 1,4% werden über längere Zeit durch intensives Rühren bei 50°C in 50 g Methylmethacrylat gelöst.

Bei Raumtemperatur werden dann
10 g Methacrylsäure,
9 g 1,4-Butandioldimethacrylat und
1 g Dibenzoylperoxid zugesetzt.

Komponente B :

Analoge Zubereitung zur Komponente A mit 1,0 g N,N'-Dimethyl-p-toluidin anstatt Dibenzoylperoxid.

Durch Vermischen von gleichen Teilen der Komponenten A und B härtet das erfindungsgemässe Material bei Raumtemperatur nach 5 bis 7 Minuten zu einem auf handelsüblichen Dentallegierungen fest haftenden, flexiblen Film aus.

Beispiel 3 :

Komponente A :

30 g eines chlorosulfonierten Polyäthylens mit einer Molekulargewichtsverteilung zwischen 20'000 und 40'000, einem Chlorgehalt von 29% und einem Schwefelgehalt von 1,4% werden, wie in Beispiel 2 beschrieben, in

25 g Methylmethacrylat und
25 g 1,1,5 Trihydro-tetrafluoropropylmethacrylat gelöst.
Bei Raumtemperatur wird die Lösung mit
10 g Methacrylsäure,
9 g Hexafluoro-1,5-pentandiol-dimethacrylat und
1 g Dibenzoylperoxid verdünnt.

Komponente B :

Analoge Zubereitung zur Komponente A mit 1,0 g N,N'-Dimethyl-p-toluidin statt Dibenzoylperoxid.

Durch Vermischen von gleichen Teilen der Komponenten A und B härtet das erfindungsgemässe Material bei Raumtemperatur nach 7 Minuten zu einem auf handelsüblichen Dentallegierungen fest haftenden, flexiblen Film aus.

Beispiel 4 :

Analoge Zubereitung gemäss Komponente A des Beispiels 3 mit Ersatz des Dibenzoylperoxids durch 1,0 g Benzildimethylketal.

Beim Bestrahlen mit Osram-Leuchtstoffröhren (Dulux S9W/18) härtet dieses Einkomponentenmaterial innerhalb von 4 Minuten zu einem auf Dentallegierungen fest haftenden, flexiblen Film aus.

Durchgeführte Vergleichsmessungen :

A) Wasseraufnahme :

Zur Prüfung der Wasseraufnahme nach der ADA-Spezification 27 wurden scheibenförmige Proben mit einer Dicke von 1 mm und einem Durchmesser von 20 mm hergestellt. Diese Proben wurden solange in einem Exsiccator mit Silicagel aufbewahrt bis der Gewichtsverlust von einem Tag auf den anderen kleiner als 0,5 mg war. Danach wurde die Probe 7 Tage in Wasser bei 37°C gelagert. Anschliessend wurde die Probe abgetrocknet und gewogen. Aus dem Gewichtsverlust und den Abmessungen der Probe wurde die Wasseraufnahme in $mg/cm^2$ Probenoberfläche berechnet.

| Beispiel Nr. | Wasseraufnahme in $mg/cm^2$ |
|---|---|
| 1 | 3,40 ± 0,60 |
| 2 | 0,89 ± 0,03 |
| 3 | 0,21 ± 0,03 |
| 4 | 0,15 ± 0,04 |

B) Abschertest :

Die Prüfung wurde mittels normierter Verfahren auf einer "Zwick-Universal-Prüfmaschine Typ 1435" bei 1 mm/min Geschwindigkeit durchgeführt.

Die als Vergleichsmaterial mitgeprüften Systeme (ISOSIT N und SILICOATING) wurden streng nach den Herstellerangaben verarbeitet.

Es wurden folgende Werte ermittelt :

- der Grundwert nach 24 Stunden Lagerung bei Raumtemperatur an der Luft,
- der Wert nach ein, drei und sechs Monaten Lagerung bei 37°C in destilliertem Wasser und
- der Wert nach einer Temperaturwechselbelastung von 4°C und 54°C bei jeweiliger Verweilzeit von 30 Sekunden.

Jeder Wert wurde als Mittelwert aus zehn Messungen errechnet. Die ermittelten Haftwerte sind in der nachfolgenden Tabelle aufgeführt.

Tabelle

Ermittelte Haftwerte in N/mm²

| Material | Grundwert | Wasserlagerung 37° 1 Monat | 3 Monate | 6 Monate | Korund (μ) Korngröße | 200 Intervalle 4° - 54°C |
|---|---|---|---|---|---|---|
| Isosit N (Vivadent) | 7,6 ± 0,8 | 6,4 | 6,1 | 5,0 | 50 | 3,4 ± 0,5 |
| Isosit N | 6,7 ± 0,9 | 5,7 | 5,7 | 4,7 | 200 | 3,5 ± 1,0 |
| Silicoating (Kulzer) | 5,6 ± 0,7 | 6,7 | 6,5 | 5,0 | 50 | 8,5 ± 1,5 |
| Silicoating | 6,2 ± 6,4 | 7,1 | 7,1 | 6,4 | 200 | 6,0 ± 0,8 |
| Beispiel 1 | 10,5 ± 1,0 | 6,7 | 5,2 | 3,9 | 50 | 4,8 ± 0,7 |
| Beispiel 1 | 10,9 ± 1,2 | 5,9 | 4,9 | 2,8 | 200 | 4,4 ± 1,1 |
| Beispiel 2 | 10,9 ± 0,3 | 10,4 | 10,0 | 9,3 | 50 | 8,8 ± 0,5 |
| Beispiel 2 | 11,2 ± 0,5 | 10,2 | 10,0 | 9,0 | 200 | 9,1 ± 0,5 |
| Beispiel 3 | 10,2 ± 0,2 | 10,7 | 10,4 | 10,5 | 50 | 9,9 ± 0,9 |
| Beispiel 3 | 10,0 ± 0,2 | 9,9 | 10,2 | 9,8 | 200 | 10,1 ± 1,1 |
| Beispiel 4 | 10,9 ± 0,7 | 11,4 | 11,4 | 11,3 | 50 | 11,0 ± 0,4 |
| Beispiel 4 | 12,0 ± 0,3 | 11,9 | 12,1 | 11,9 | 200 | 11,7 ± 0,4 |

Bemerkung: Das Ansteigen einzelner Haftwerte trotz Belastung mit z.B. Temperaturintervallen wird als Nachhärtung interpretiert.

## Ansprüche

1. Haftvermittler basierend auf einem Acryl-, Methacryl- oder Vinyl-Gruppen aufweisenden flüssigen Monomer, Monomerengemisch oder Präpolymer mit einem Zusatz von Acryl- oder Methacrylsäure als Grundgemisch, dadurch gekennzeichnet, dass das Grundgemisch zusätzlich

A) ein halogensulfoniertes Polyolefin ; und

B) ein halogeniertes Acrylat oder Methacrylat enthält.

2. Haftvermittler nach Anspruch 1, dadurch gekennzeichnet, dass das halogensulfonierte Polyolefin ein chlorosulfoniertes Polyolefin ist, vorzugsweise vom Polyäthylen- oder Polypropylen-Typ.

3. Haftvermittler nach Anspruch 2, dadurch gekennzeichnet, dass das halogensulfonierte Polyolefin ein chlorosulfoniertes Polyäthylen mit einer Molekulargewichtsverteilung zwischen 20'000 und 40'000, einem Chlorgehalt zwischen 29-43 Gew.% und einem Schwefelgehalt zwischen 1,0-1,5 Gew.% ist.

4. Haftvermittler nach einem der Ansprüche 1 bis 3, dadurch gekennzeichnet, dass das halogenierte Acrylat oder Methacrylat ein fluoriertes mono- oder polyfunktionelles Acrylat oder Methacrylat ist.

5. Verwendung des Haftvermittlers nach einem der Ansprüche 1 bis 4, zur Herstellung eines Metall-Kunststoff-Verbundsystems in der Zahntechnik, insbesondere von Kunststoffverblendungen und Geschiebebefestigungen.

6. Verwendung des Haftvermittlers nach einem der Ansprüche 1 bis 4, zur Herstellung eines Keramik-Kunststoff-Verbundsystems in der Zahntechnik, insbesondere zur Reparatur von abgeplatzten Keramikfacetten.

## Claims

1. Bonding agent based on a basic mixture of liquid monomers, monomeric mixtures or prepolymers containing acrylic, methacrylic or vinyl groups, with an addition of acrylic or methacrylic acid, said basic mixture additionally containing

A) a halogen-sulfonated polyolefin, and

B) a halogenated acrylate or methacrylate.

2. Bonding agent according to claim 1, characterized in that said halogen-sulfonated polyolefin is a chloro-sulfonated polyolefin, preferably of the polyethylene or polypropylene type.

3. Bonding agent according to claim 2, characterized in that said halogen-sulfonated polyolefin is a chloro-sulfonated polyethylene with a molecular weight distribution between 20'000 and 40'000, a chlorine content between 29-43 weight percent and a sulphur content between 1,0-1,5 weight percent.

4. Bonding agent according to one of the claims 1 to 3, characterized in that said halogenated acrylate or methacrylate is a fluorinated mono- or polyfunctional acrylate or methacrylate.

5. Use of the bonding agent according to one of the claims 1 to 4 for producing a metal-plastics connection in dental technology, in particular metal-plastics veneerings and dental attachments.

6. Use of the bonding agent according to one of the claims 1 to 4 for producing a ceramic-plastics connection in dental technology, in particular for the repairing of chipped off ceramic facets.

## Revendications

1. Agent adhésif fondé sur un mélange de base, qui se compose de monomères, mélanges de monomères, ou de prépolymères, liquides, présentant des radicaux acryle, méthacryle ou vinyle et d'une addition d'acide acrylique ou méthacrylique, qui contient complémentairement encore

A) une polyoléfine halogénosulfonée et

B) un acrylate ou méthacrylate, halogéné.

2. Agent adhésif suivant la revendication 1, caractérisé en ce que la polyoléfine halogénosulfonée est une polyoléfine chlorosulfonée, préférablement du type polyéthylène ou polypropylène.

3. Agent adhésif suivant la revendication 2, caractérisé en ce que la polyoléfine halogénosulfonée est une polyéthylène chlorosulfonée avec une distribution du poids moléculaire entre 20'000 et 40'000, une teneur en chlore entre 29-43 pour-cent en poids et une teneur en soufre entre 1,0-1,5 pour-cent en poids.

4. Agent adhésif suivant l'une des revendications 1 à 3, caractérisé en ce que l'acrylate ou le méthacrylate, halogéné, est un acrylate ou méthacrylate, mono- ou polyfonctionnel, fluoré.